# EUROPEAN PATENT APPLICATION

(11) **EP 4 557 619 A1**
(43) Date of publication of application: **21.05.2025**
(21) Application number: 24213079.7
(22) Date of filing: 14.11.2024
(51) Int. Cl.: H03M 1/18, H03M 1/46, A61B 5/1455, G01N 33/49

(54) **LOW LATENCY AUTOMATIC CROSS TALK CANCELLATION FOR LED-BASED SENSORS**

(30) Priority: 14.11.2023 US 202363598857 P; 12.11.2024 US 202418945092
(71) Applicant: Analog Devices, Inc., Wilmington, MA 01887 (US)
(72) Inventor: YANG, Yaohua, Wilmington, 01887 (US); MCCARROLL, Benjamin John, Wilmington, 01887 (US); PARK, Min, Wilmington, 01887 (US)
(74) Representative: Wallin, Nicholas James

(57) **Abstract**

Aspects of the present disclosure provide methods and apparatuses for operating an analog-to-digital converter. A method in accordance with an aspect of the present disclosure may comprise initializing a digital-to-analog converter (DAC) value of a DAC, determining whether an analog-to-digital converter (ADC) operates within a predetermined range based on an input to the DAC, initiating a conversion at a first ADC resolution when the ADC is operating within the predetermined range, and incrementally changing the DAC value when the ADC is not operating within the predetermined range.

## Description

### CROSS-REFERENCE TO RELATED APPLICATIONS

This application claims priority to and the benefit of U.S. Non-Provisional Patent Application No. 18/945,092, filed November 12, 2024 which claims the benefit of U.S. Provisional Patent Application No. 63/598,857, filed on November 14, 2023, the contents of each application are hereby incorporated herein by reference in their entireties.

### BACKGROUND

### Technical Field

The present disclosure generally relates to control circuits for optical devices. More particularly, the present disclosure relates to systems and methods for LED-based biometric optical diagnostic sensors.

### Introduction

The miniaturization of electronic diagnostic sensors has allowed for their integration into portable monitoring devices and smartphones. Such lightweight diagnostic sensors provide a means for non-intrusive detection and measurement of bodily conditions, such as vital signs, e.g., heart rate, oxygen saturation level, heart rate variability, heart valve closure timing, and other useful information to aid in monitoring physical activity and fitness, and even the diagnosis of health conditions.

Noninvasive biometric optical sensors, such as pulse oximeter sensors, utilize optical red and IR signals reflected from blood vessels under the skin to measure, e.g., peripheral oxygen saturation (SpO2) levels in a person's blood. Pulse oximeter sensors are used as photoplethysmography (PPG) sensors that monitor periodically changing blood volume pulsations or perfusion in the skin. In addition, the detected optical signals may be used to determine a person's heart rate. Measurements commonly involve shining green light and red light generated by LED light sources onto the person's skin and measuring light reflected from the skin using a photodiode that is typically located within the same sensor. The gathered information, such as the AC component of the light, can then be used to calculate heart rate and determine the concentration of oxygen in the user's blood vessels under the skin, etc.

One major concern of LED-based optical sensors is cross talk, i.e., a portion of the LED light that directly enters the photodiode without being reflected from the person's skin, due to the limitations of the sensor's optical design. Cross talk reduces the dynamic range of the receiver analog front end (AFE) and may in some instances be large enough to saturate the receiver's small-dynamic-range ADC.

Some existing control mechanisms utilize AFEs allow a user to at least partially cancel the effects of cross talk by using an on-chip DAC. In such designs, the user, with the help of an off-chip microcontroller, adjusts a DAC value through reading an ADC output and feedback algorithm. One major drawback of such approaches is latency associated with the off-chip algorithm, which can be as high as 8 AFE samples. Combined with the generally low sample rate of AFEs aimed at minimizing power consumption, the latency of the feedback loop can be as long as a fraction of a second, which is unacceptable for many applications.

Accordingly, what is needed are systems and methods that not only reduce unwanted crosstalk, latency, and power consumption of LED-based biometric sensors and, thus, improve overall sensor performance, while maintaining a high level of accuracy.

### SUMMARY

The following presents a simplified summary of one or more aspects in order to provide a basic understanding of such aspects. This summary is not an extensive overview of all contemplated aspects, and is intended to neither identify key or critical elements of all aspects nor delineate the scope of any or all aspects. Its sole purpose is to present some concepts of one or more aspects in a simplified form as a prelude to the more detailed description that is presented later.

A method in accordance with an aspect of the present disclosure may comprise initializing a digital-to-analog converter (DAC) value of a DAC, determining whether an analog-to-digital converter (ADC) operates within a predetermined range based on an input to the DAC, initiating a conversion at a first ADC resolution when the ADC is operating within the predetermined range, and incrementally changing the DAC value when the ADC is not operating within the predetermined range.

Such a method further optionally includes changing the DAC value including changing the DAC current, incrementally changing the DAC value comprising increasing the DAC value, the input to the DAC being a photodiode current, the ADC being a successive approximation register (SAR) based ADC, the DAC value being based on a number of bits of the SAR based ADC, the DAC value including a settling time of a light-emitting diode, and the DAC value including a conversion time of the ADC.

An apparatus in accordance with an aspect of the present disclosure may comprise a digital-to-analog converter (DAC) having a DAC value, a logic circuit coupled to the DAC, an analog-to-digital converter (ADC) coupled to the DAC and the logic circuit, the ADC operating in a predetermined range based on an input to the DAC, and a comparator circuit coupled to the logic circuit, the ADC, and the DAC, wherein the logic circuit and the comparator circuit initiate a conversion at a first ADC resolution when the ADC is operating within the predetermined range and incrementally change the DAC value until the ADC is operating within the predetermined range.

Such an apparatus may further optionally include changing the DAC value comprising changing the DAC current, incrementally changing the DAC value comprising increasing the DAC value, the input to the DAC being a photodiode current, the ADC being a successive approximation register (SAR) based ADC, the DAC value being based on a number of bits of the SAR based ADC, the DAC value including a settling time of a light-emitting diode, and the DAC value including a conversion time of the ADC.

To the accomplishment of the foregoing and related ends, the one or more aspects comprise the features hereinafter fully described and particularly pointed out in the claims. The following description and the annexed drawings set forth in detail certain illustrative features of the one or more aspects. These features are indicative, however, of but a few of the various ways in which the principles of various aspects may be employed, and this description is intended to include all such aspects and their equivalents.

### BRIEF DESCRIPTION OF THE DRAWINGS

References will be made to an aspect of the present disclosure of the disclosure, examples of which may be illustrated in the accompanying figures. These figures are intended to be illustrative, not limiting. Although the invention is generally described in the context of these an aspect of the present disclosure, it should be understood that it is not intended to limit the scope of the disclosure to these particular an aspect of the present disclosure. Items in the figures are not to scale unless specifically noted.
FIG. 1A illustrates an LED-based optical sensor circuit for monitoring vital signs utilizing a microcontroller in accordance with an aspect of the present disclosure.
FIG. 1B illustrates a sensor arrangement in accordance with an aspect of the present disclosure.
FIG. 2 illustrates an input-output relationship for an ADC, such as the ADC used in the sensor circuit shown in FIG. 1A, in accordance with an aspect of the present disclosure.
FIG. 3A is a block diagram of a vital signs monitoring system that comprises diagnostic LED-based biometric optical sensors in accordance with an aspect of the present disclosure.
FIG. 3B illustrates a timing diagram in accordance with an aspect of the present disclosure.
FIG. 4A illustrates an example input-output relationship between current DAC input and a photodiode current in accordance with an aspect of the present disclosure.
FIG. 4B illustrates an example input-output relationship between ADC input and photodiode current in accordance with an aspect of the present disclosure.
FIG. 5 is a flowchart of an illustrative process for low-latency automatic cross talk cancellation in accordance with an aspect of the present disclosure.
FIG. 6 illustrates a process flow in accordance with an aspect of the present disclosure.

### DETAILED DESCRIPTION

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components are shown in block diagram form in order to avoid obscuring such concepts.

### Overview

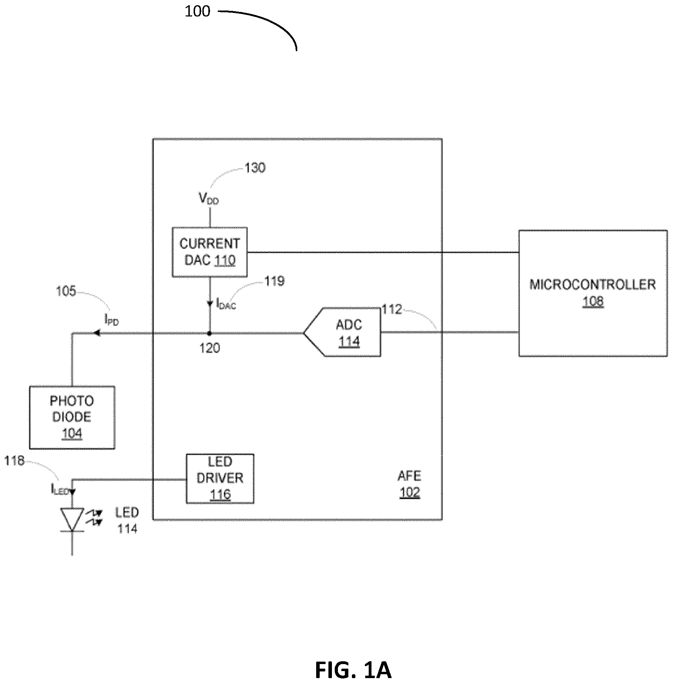

FIG. 1A illustrates an LED-based optical sensor circuit for monitoring vital signs utilizing a microcontroller in accordance with an aspect of the present disclosure.

FIG. 1A illustrates a prior art light-emitting diode (LED) based optical sensor circuit for monitoring vital signs utilizing a microcontroller. Circuit 100 comprises Analog Front End (AFE) 102, photodiode 104, LED 114, and microcontroller 108. AFE 102, in turn, comprises LED driver 116, ADC 106, current DAC 110. Typically, LED 114 and photodiode 104 are part of an LED-based optical sensor, such as a heart rate sensor.

In operation, photodiode 104 detects light that is reflected from part of a person's body, e.g., skin, which has been illuminated by nearby LED 114. LED driver 116 controls the intensity of the light emitted by LED 114. Based on the amount of light that photodiode 104 detects, photodiode 104 generates a continuous analog current 105 that is then converted by ADC 106 into digital output signal 112, which is further processed by microcontroller 108.

The analog current 105 that photodiode 104 generates comprises an AC component that has a certain frequency (e.g., 20 Hz). It is typically this AC component that carries useful information that is related to, e.g., heart rate, such as a blood volume pulsation, and other data useful in determining vital signs or other physiological states of a person. The analog current 105 that ADC 106 receives as input signal also contains a relatively large DC portion mainly caused by cross talk, i.e., extraneous light sources, such as ambient light or scattered light, that directly or indirectly enters photodiode 104 without being reflected from blood vessels and, thus, does not contain the sought after information.

Since analog current 105 from photodiode 104 is typically proportional to the current flowing through LED 114, increasing the LED current 118, i.e., the ADC input signal, increases not only the AC component but also the unwanted DC component. Therefore, increasing the input signal range of ADC 106 by shining more LED light onto the body part to increase the amount of light reflected from the body and, in turn, increase analog current 105 does not automatically increase resolution. Instead, the additional light will eventually cause ADC 106 to saturate, thus, rendering the attempt futile.

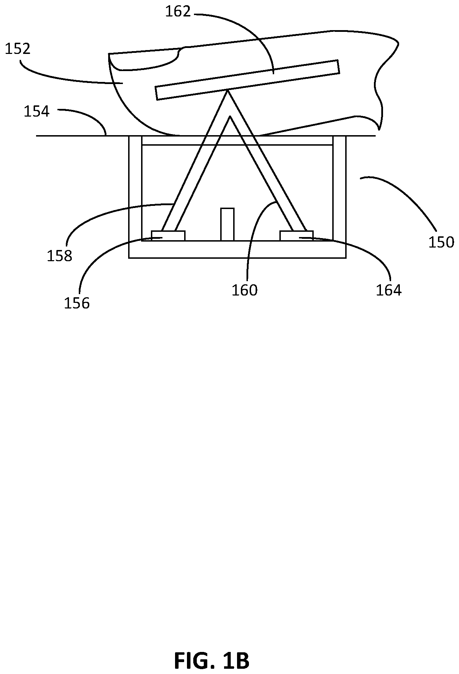

FIG. 1B illustrates a sensor arrangement in accordance with an aspect of the present disclosure.

Sensor 150 is shown with a subject 152, e.g., a human finger, placed on surface 154 of sensor 150. Within sensor 150, LED 156, which may be similar to LED 114, shines light 158 and 160 onto subject 152. Some of the light from LED 156, shown as light 158, reaches artery 162 in subject 152, while other light, shown as light 160, is reflected from other parts of subject 152. Photodiode 164, which may be similar to photodiode 104, receives the light 158 and 160 after being reflected from the various portions of subject 152.

Light 158 and light 160 may be in various portions of the spectrum such that the light received at photodiode 164 is indicative of what portion of subject 152 is being sensed. For example, light in one portion of the spectrum, e.g., the infrared portion, may be used to determine the artery 162 flow, while light in another portion, e.g., the red, green, or blue portions of the visible spectrum, may be used to determine other parameters such as skin thickness, skin absorption, and/or other parameters.

As photodiode 164 receives more or less light 158, e.g., the light that reaches artery 162, the amount of oxygen present in the blood flowing in artery 162 can be determined.

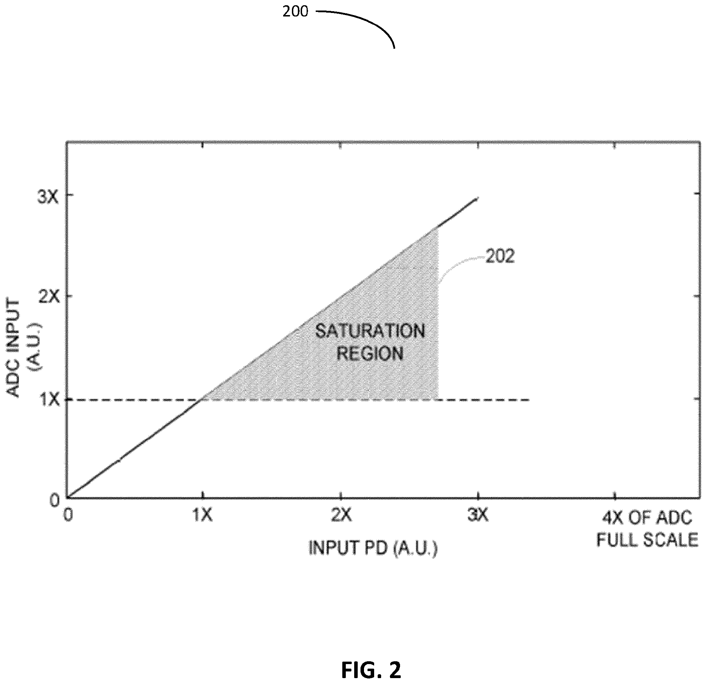

FIG. 2 shows an input-output relationship for an existing ADC, such as the ADC used in the sensor circuit shown in FIG. 1A in accordance with an aspect of the present disclosure.

FIG. 2 shows an input-output relationship 200 for an ADC, such as the ADC used in the LED-based optical sensor circuit in FIG. 1A. As can be seen in FIG. 2, once photodiode input current becomes so large that the ADC is forced to operate at full scale, the ADC reaches its limit and ADC enters saturation region 202, which renders the ADC unsuitable for its intended purpose.

An additional major drawback stems from the fact that motion artifacts, pressure changes, etc., are not constant but rather drift over time, in effect, presenting a moving target for the ADC that makes it difficult to zoom into a final value within a reasonably short settling time, i.e., before the to-be-sampled value may have already changed. Therefore, instead of successfully converging after a certain number of cycles, this may cause sampling to fall out of range.

Returning to FIG. 1A, in order to reduce the unwanted DC component of the photo diode current, certain designs, such as that shown in FIG. 1A, employ a current source, such as programmable current DAC 110 to impress a current (e.g., from VDD 130 into node 120) to cancel at least some portion of the DC component in analog current 105 that flows into ADC 106. This allows a user to reap the benefits of an increased LED current 118 without stepping out of ADC range and saturating ADC 106, even if some of the LED light circumvents the to be illuminated body part as cross talk.

AFE 102 in FIG. 1A communicates ADC's digital output signal 112, e.g., in the form of an ADC count, to microcontroller 108 that extracts heart rate data and possibly other useful information. Microcontroller 108 can also monitor whether ADC 106 has been saturated. For example, once microcontroller 108, in response to obtaining ADC digital output signal 112 value, determines that ADC 106 is saturated, it can conclude that analog current 105 from photodiode 104 is too high and, thus, increase the DAC bit value of DAC 110 to increase DAC current 119 such as to counteract (i.e., cancel) the cross talk. Likewise, if microcontroller 108 concludes that analog current 105 is below a desired range, microcontroller 108 can decrease DAC current 119 to prevent canceling too much of analog current 105 and cause ADC to under-range.

Oftentimes, microcontroller 108 typically accomplishes this by using a relatively slow feedback loop (not shown in FIG. 1A) that in existing designs is adjusted at most once per sample / reading. In addition, due to power considerations, the sample rate is typically kept relatively low (e.g., about 25Hz), such that ADC 106 reads at a relatively slow rate (e.g., every 40ms) to keep the ADC current within range. However, this approach results in a relatively long latency that negatively affects certain applications.

Therefore, it would be desirable to have systems and methods that provide near-instantaneous conversion rates to ensure that every sample remains within its range and to compensate for unwanted crosstalk in LED-based sensor, ideally, reducing latency and power consumption at the same time without sacrificing sensor accuracy.

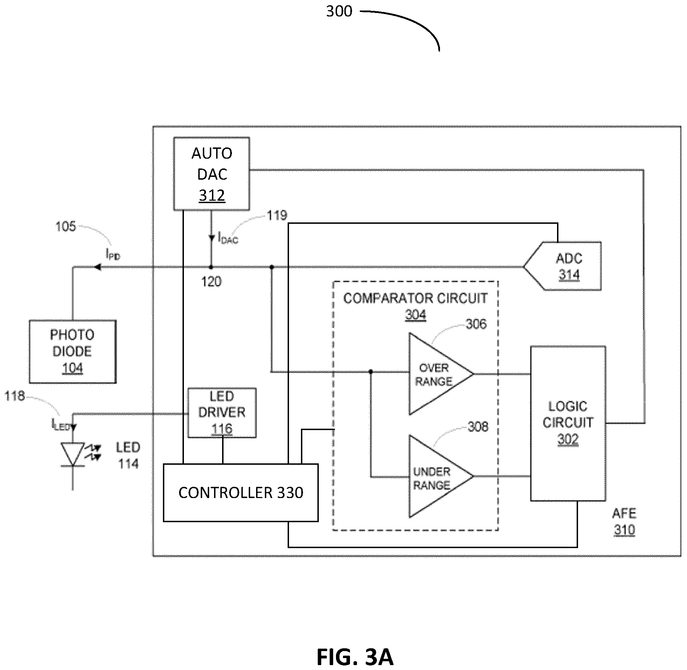

FIG. 3A is a block diagram of a vital signs monitoring system that comprises diagnostic LED-based biometric optical sensors in accordance with an aspect of the present disclosure.

FIG. 3A is a block diagram of a vital signs monitoring system that comprises diagnostic LED-based biometric optical sensors according to various aspects of the present disclosure. For clarity, components similar to those shown in FIG. 1A are labeled in the same manner. For purposes of brevity, a description or their function is not repeated here. In an aspect of the present disclosure, system 300 may comprise AFE 310, photodiode 104, and LED 114. AFE 310 may comprise on-chip logic circuit 302, comparator circuit 304, LED driver 116, ADC 314, and auto-DAC 312 that may be implemented as an on-chip auto-DAC. AFE 310 may also include a controller 330 that is coupled to LED driver 116, auto-DAC 312, comparator circuit 304, logic circuit 302, and ADC 314. Controller 330 may be a microcontroller, or other processor unit as desired without departing from the scope of the present disclosure.

As depicted in FIG. 3A, comparator circuit 304 may comprise overrange comparator 306 and overrange comparator 308. LED 114 and photodiode 104 may be parts of an LED-based biometric optical sensor, such as a SpO2 sensor or a PPG sensor. System 300 is a low-latency system that may automatically track and cancel cross talk latency by using logic circuit 302 and on-chip auto-DAC 312 to automatically obtain the optimal cross talk cancellation DAC code on chip.

In operation, system 300 may commence a crosstalk cancellation by setting auto-DAC 312 to zero. In an aspect of the present disclosure, if ADC 314 is in within its regular operation range, no further action needs to be taken, and system 300 may perform a conversion at full resolution. In an aspect of the present disclosure, if overrange comparator 306 in comparator circuit 304 detects that ADC 314 operates above a predetermined range, the DAC value may be incrementally increased, e.g., by setting a subsequent DAC value to a predetermined count to increase the DAC current and, thus, cancel more of the cross talk. Conversely, if underrange comparator 308 in comparator circuit 304 detects that ADC 314 operates below a desired range, the DAC value may be incrementally decreased to decrease the DAC current and, thus, cancel less of the cross talk.

Alternatively, if ADC 314 operates within a regular operating range, ADC 314 may perform a conversion at full resolution.

In an aspect of the present disclosure, auto-DAC 312 may be implemented as an on-chip auto-DAC that generates output values representative of an amount of cross talk that AFE 310 cancels within a given time period. In an aspect of the present disclosure, the output values may be made available, e.g., via a FIFO (not shown), to a user to enable the user to estimate the amount of cross talk AFE 310 is canceling. Advantageously, such on-chip cross talk cancellation aspects of the present disclosure may reduce and/or eliminate the need for external firmware control loops and delay (e.g., at least 1 sample delay), thus, providing an accurate digital representation of the photo diode input free from saturation effects.

In addition, in an aspect of the present disclosure, the need for user input may be eliminated. However, this is not intended as limitation on the scope of the disclosure since in off-chip cross talk cancellation aspects, auto-DAC 312 may receive user input, e.g., from a micro-controller (not shown) that may be in control of the user, such that auto-DAC 312 can cancel cross talk based on, e.g., a user-defined algorithm.

The present disclosure may be implemented using additional components, such as noise filtering elements, etc., to support various functions of system 300 according to the objectives of the disclosure. For example, the AFE 310 is not limited to the constructional detail shown there or described in the accompanying text. Additional components that may aid in implementing certain aspects of the present disclosure may comprise amplifiers, sample-and-hold circuits, and so on, without departing from the scope of the present disclosure.

In an aspect of the present disclosure, a Successive Approximation Register (SAR)-based ADC topology may be utilized to acquire high speed data. A SAR-based ADC may be any type of ADC that may perform conversion by converting a continuous analog input signal into a discrete digital output signal by using a binary search through all quantization levels. For example, a 2-step low-power SAR-based ADC may use any known method or search algorithm before, ultimately, converging on a digital value. In an aspect of the present disclosure, the SAR-based ADC may, e.g., in a SAR timing phase, perform a binary search that successively converts a continuous analog input signal into a discrete digital output signal such as to enable system 300 to determine an optimal DAC code.

As a person of skill in the art will appreciate, an ADC that samples values as a 20-bit ADC, i.e., performing a 20-bit resolution conversion, will be slower than a 4-bit SAR-based ADC. Therefore, instead of performing a time consuming and power consuming 20-bit resolution conversion, various aspects of the present disclosure take relatively fast and low-resolution samples to significantly reduce latency. In an aspect of the present disclosure, during the SAR timing phase, the SAR-based ADC may operate in a low-resolution mode, thus, significantly reducing the amount of conversion time.

Further, a number of sub-steps may be performed within the SAR timing phase, e.g., each sub-step having a given duration of about 3µ sec, such that if a SAR-based ADC applies four sub-steps, the SAR timing phase would last 12µsec. In other words, an LED Pulse width and LED settling time would increase by about 10µs. In an aspect of the present disclosure, during this time, the ADC may perform a number of low-resolution ADC conversions to provide the decisions for the SAR logic. At the end of the 10µs SAR timing phase, a correct auto DAC value may be chosen such that the ADC will not exceed its regular range of operation.

Since an optimal DAC value may be obtained in the SAR timing phase, a cross talk cancellation DAC value may be applied to the ADC input for each sample to ensure that ADC 314 does not saturate for any given sample, even if the cross talk should vary from sample to sample.

In an aspect of the present disclosure, the conversion may comprise a second phase, e.g., a regular ADC conversion phase, in which the SAR-based ADC may converge to a final value to perform a full resolution conversion. In this manner the SAR timing phase, in effect, obviates the control feedback loop in FIG. 1A.

In an aspect of the present disclosure, since the total conversion time may be in the µsec range, the to-be-measured value-and the illuminated body part-are much less likely to move around during this relatively small time period before the value is sampled. Advantageously, this ensures a high level of accuracy. In comparison to existing designs that exhibit latencies on the order of a fraction of a second, the latency of various aspects of the present disclosure presented is on the order of µs. In addition, advantageously power consumption greatly reduced when compared to existing designs that employ relatively power-hungry microcontrollers, such as that shown in FIG. 1A.

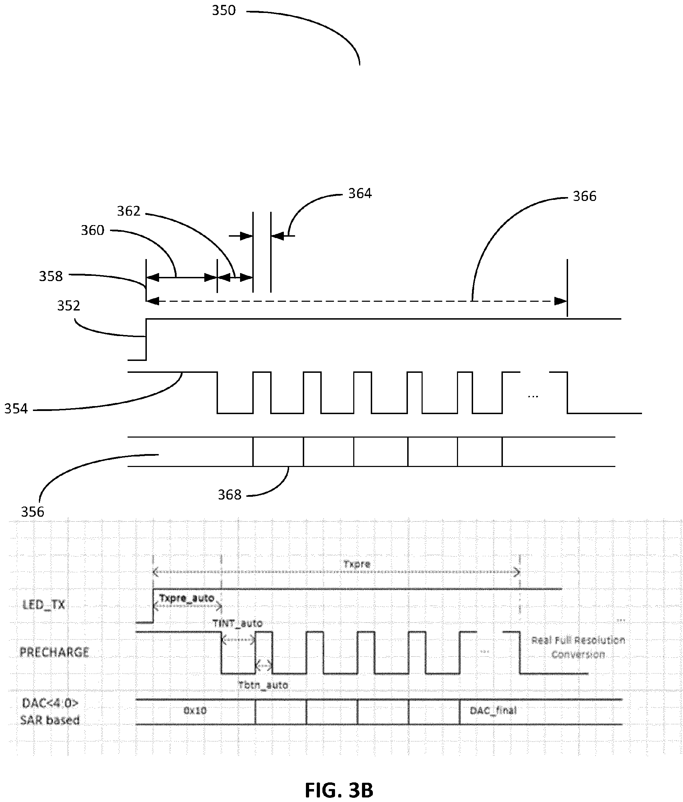

FIG. 3B illustrates a timing diagram in accordance with an aspect of the present disclosure.

Diagram 350 illustrates LED transmit 352, precharge 354, and DAC 356. DAC 356 may be auto-DAC 312 as shown in FIG. 3A. The LED transmit 352 is turned on at time 358 and allowed to settle for a time period 360. Precharge 354 goes low after time period 360 for interval 362, and returns to a high state after between period 364. The changes for precharge 354 continue for the total time period 366.

DAC 356 may be based on the SAR and the number of bits in the SAR for determining total time period 366. As shown in FIG. 3B, DAC 356 may be based on a five-bit SAR, with each bit 368 shown as part of DAC 356.

For example, and not by way of limitation, the total time period 366 may be determined by adding time period 360 (the settling period of the LED), and a multiple of interval 362 and between period 364, where the multiple is the number of bits in the SAR. An additional amount of time may also be added to total time period 366 for the DAC to produce the conversion.

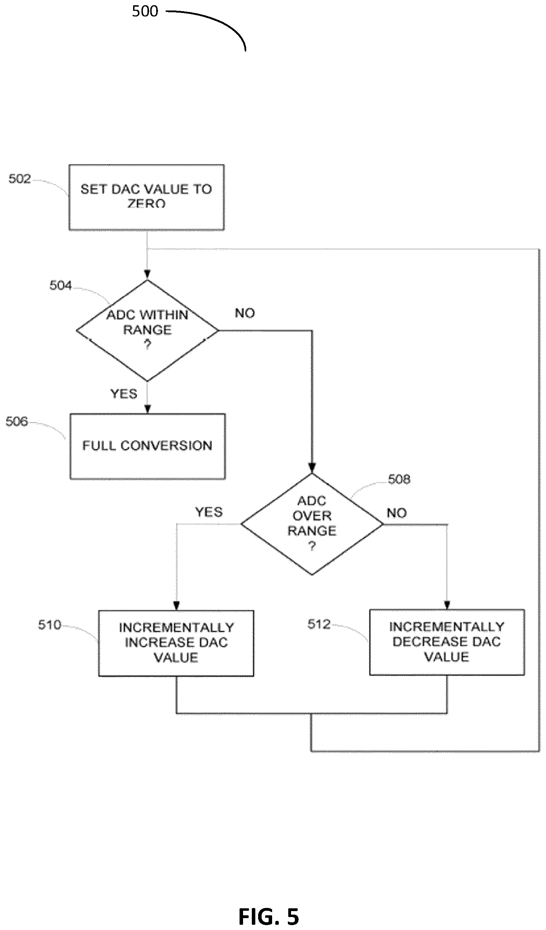

In the example shown in FIG. 3B, time period 360 may be 4 microseconds (us). Each bit in the SAR may be 3 us, which is the sum of interval 362 and between period 364. Since five bits 368 are shown in FIG. 3B, 5 x 3us = 15us are added to the 4us of time period 360. A 0.2us DAC conversion time is added to this total, giving 19.2 us for the total amount of time needed to produce an in-scale DAC conversion in accordance with an aspect of the present disclosure. Other amounts of time for each bit 368, a different number of bits 368, the time period 360 (the settling time of the LED transmit 352), and the DAC conversion time may be used without departing from the scope of the present disclosure. Further, in an aspect of the present disclosure, the total time period 366 may be rounded up to a particular number, e.g., 19.5us or 20us in the present example, or other amounts, to reduce power draw, to match sampling rates, or for other purposes, without departing from the scope of the present disclosure.

In an aspect of the present disclosure, ADC 314 may have a large full-scale range, e.g., 32 microamps (uA), to reduce and/or avoid changes to the auto-DAC 312 code. However, other algorithms, such as a full-scale range of 4, 8, and/or 16uA, may be employed without departing from the scope of the present disclosure.

Whatever the full-scale range of the ADC 314 is, the SAR threshold may be adjusted to be centered about the middle of the scale, so there is the largest available margin before ADC 314 saturates. In subsequent frames (i.e., total time period 366), the previous DAC value is maintained unless the ADC 314 output gets close to a predetermined threshold close to the upper/lower bounds of the ADC 314 full scale measurement. If the ADC 314 output enters this threshold, the next total time period 366 may re-calibrate the ADC full-scale by using the auto-DAC 312 to recalibrate the full-scale of the ADC 314. The predetermined threshold may be a percentage of full scale, e.g., 10%-90% of full scale. Other boundaries, e.g., 15% to 85%, etc., may be used without departing from the scope of the present disclosure.

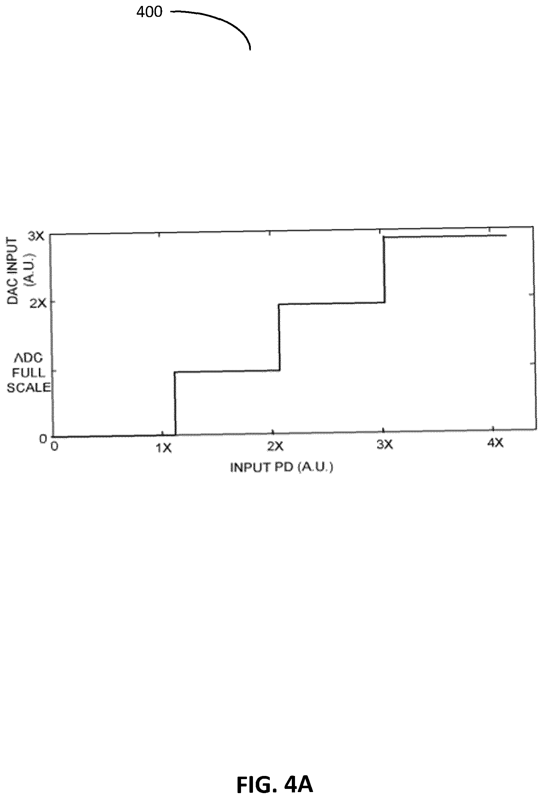

FIG. 4A illustrates an example input-output relationship between current DAC input and a photodiode current in accordance with an aspect of the present disclosure.

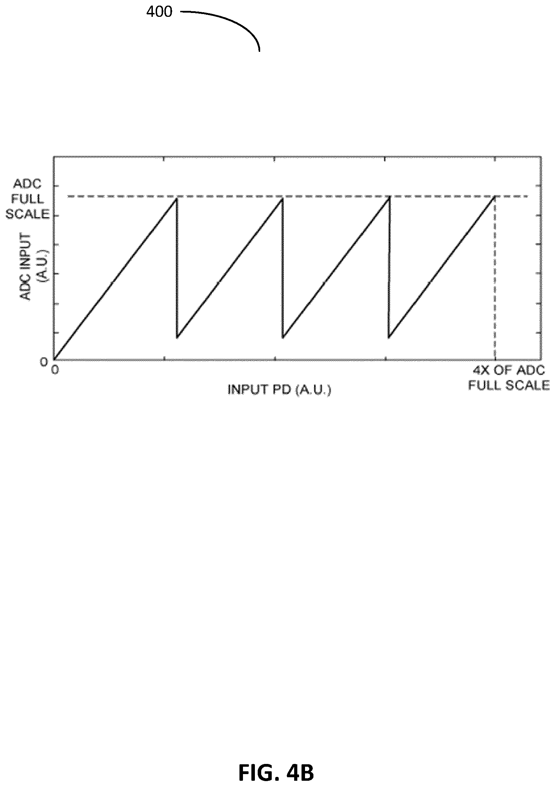

FIG. 4A illustrates an example input-output relationship between current DAC input values and a photodiode input current, according to various aspects of the present disclosure. In an aspect of the present disclosure, the electrical current (also referred to as "current") DAC input may assume values that stepwise increase, each step being aligned with the ADC reaching a value near its full operating range. As depicted in FIG. 4A the DAC current increases stepwise with the input photodiode current to cancel cross talk. As illustrated in the example input-output relationship between ADC input values and photodiode input current in FIG. 4B, in an aspect of the present disclosure, each time the ADC input reaches a value near its full operating range, the ADC may obtain a counter current from the DAC that reduces the ADC input current. Advantageously, this allows the ADC input current to remain within a proper operating range, irrespective of the photodiode current. In an aspect of the present disclosure, this is accomplished by applying low-latency automatic cross talk cancellation discussed with reference to FIG. 3A.

FIG. 4B illustrates an example input-output relationship between ADC input and photodiode current in accordance with an aspect of the present disclosure.

In an aspect of the present disclosure, the current DAC values may be stepwise subtracted from the ADC input current to obtain the result in FIG. 4B, i.e., without the ADC running the risk of saturating.

FIG. 5 is a flowchart of an illustrative process for low-latency automatic cross talk cancellation in accordance with an aspect of the present disclosure.

FIG. 5 is a flowchart of an illustrative process for low-latency automatic cross talk cancellation in accordance with various aspects of the present disclosure. Process 500 may begin, at step 502, by initializing a DAC value, e.g., by setting it to zero. At step 504, it is decided whether ADC is within range. If so, at step 506, a conversion may be performed at full resolution. Otherwise, if ADC is not within range at step 504, is determined, at step 508, whether the ADC operates over range. If so, process 500 enters step 510 and the DAC value may be incrementally increased to increase the DAC current and, thus, cancel more of the cross talk. Otherwise, if ADC operates under range, the DAC value may be incrementally decreased, at step 512, to decrease the DAC current and cancel less of the cross talk.

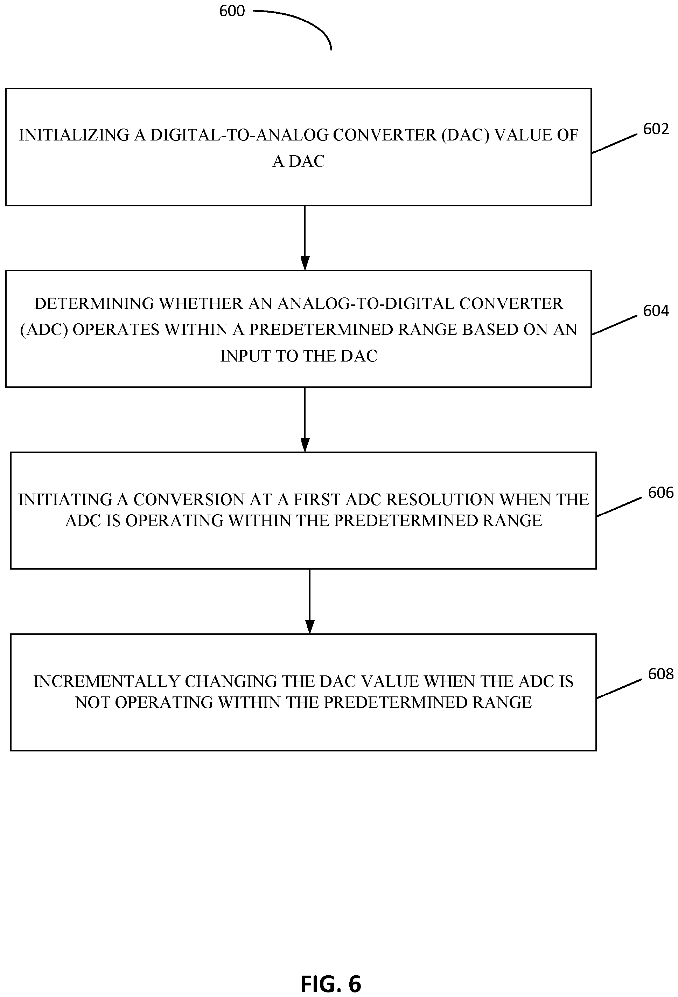

FIG. 6 illustrates a process flow in accordance with an aspect of the present disclosure.

Flow 600 may include block 602, which illustrates initializing a digital-to-analog converter (DAC) value of a DAC.

Block 604 illustrates determining whether an analog-to-digital converter (ADC) operates within a predetermined range based on an input to the DAC.

Block 606 illustrates initiating a conversion at a first ADC resolution when the ADC is operating within the predetermined range.

Block 608 illustrates incrementally changing the DAC value when the ADC is not operating within the predetermined range.

The present disclosure may be implemented as one or more of the following clauses or aspects. The present disclosure is not limited to the following clauses or aspects, and the scope of the clauses or aspects may include anything described herein. Further, although the claims and clauses presented herein may be drafted in single-dependency format, it is to be understood that any claim or clause may depend on any preceding claim, clause, technique, or aspect of the same type without departing from the scope of the present disclosure.

Clause 1. A method comprising: initializing a digital-to-analog converter (DAC) value of a DAC; determining whether an analog-to-digital converter (ADC) operates within a predetermined range based on an input to the DAC; initiating a conversion at a first ADC resolution when the ADC is operating within the predetermined range; and incrementally changing the DAC value when the ADC is not operating within the predetermined range.

Clause 2. The method of clause 1, wherein changing the DAC value changes a DAC current.

Clause 3. The method of clause 1 or 2, wherein incrementally changing the DAC value comprises increasing the DAC value.

Clause 4. The method of any of clauses 1-3, wherein the input to the DAC is a photodiode current.

Clause 5. The method of any of clauses 1-4, wherein the ADC is a successive approximation register (SAR) based ADC.

Clause 6. The method of any of clauses 1-5, wherein the DAC value is based on a number of bits of the SAR based ADC.

Clause 7. The method of any of clauses 1-6, wherein the DAC value includes a settling time of a light-emitting diode.

Clause 8. The method of any of clauses 1-7, wherein the DAC value includes a conversion time of the ADC.

Clause 9. An apparatus comprising: a digital-to-analog converter (DAC) having a DAC value; a logic circuit coupled to the DAC; an analog-to-digital converter (ADC) coupled to the DAC and the logic circuit, the ADC operating in a predetermined range based on an input to the DAC; and a comparator circuit coupled to the logic circuit, the ADC, and the DAC, wherein the logic circuit and the comparator circuit initiate a conversion at a first ADC resolution when the ADC is operating within the predetermined range and incrementally change the DAC value when the ADC is not operating within the predetermined range.

Clause 10. The apparatus of clause 9, wherein changing the DAC value changes a DAC current.

Clause 11. The apparatus of any of clauses 9-10, wherein incrementally changing the DAC value comprises increasing the DAC value.

Clause 12. The apparatus of any of clauses 9-11, wherein the input to the DAC is a photodiode current.

Clause 13. The apparatus of any of clauses 9-12, wherein the ADC is a successive approximation register (SAR) based ADC.

Clause 14. The apparatus of any of clauses 9-13, wherein the DAC value is based on a number of bits of the SAR based ADC.

Clause 15. The apparatus of any of clauses 9-14, wherein the DAC value includes a settling time of a light-emitting diode.

Clause 16. The apparatus of any of clauses 9-15, wherein the DAC value includes a conversion time of the ADC.

The previous description is provided to enable any person skilled in the art to practice the various aspects described herein. Various modifications to the exemplary aspects and aspects presented throughout this disclosure will be readily apparent to those skilled in the art, and the concepts disclosed herein may be applied in other contexts and for different purposes. Thus, the claims are not intended to be limited to the exemplary aspects presented throughout the disclosure, but are to be accorded the full scope consistent with the language claims. All structural and functional equivalents to the elements of the exemplary aspects described throughout this disclosure that are known or later come to be known to those of ordinary skill in the art are intended to be encompassed by the claims. Moreover, nothing disclosed herein is intended to be dedicated to the public regardless of whether such disclosure is explicitly recited in the claims. No claim element is to be construed under the provisions of 35 U.S.C. §112(f), or analogous law in applicable jurisdictions, unless the element is expressly recited using the phrase "means for" or, in the case of a method claim, the element is recited using the phrase "step for."

## Claims

1. A method comprising:
initializing a digital-to-analog converter (DAC) value of a DAC;
determining whether an analog-to-digital converter (ADC) operates within a predetermined range based on an input to the DAC;
initiating a conversion at a first ADC resolution when the ADC is operating within the predetermined range; and
incrementally changing the DAC value when the ADC is not operating within the predetermined range.

2. The method of claim 1, wherein changing the DAC value changes a DAC current.

3. The method of claims 1 or 2, wherein incrementally changing the DAC value comprises increasing the DAC value.

4. The method of any of claims 1 to 3, wherein the input to the DAC is a photodiode current.

5. The method of claim 4, wherein the ADC is a successive approximation register (SAR) based ADC.

6. The method of claim 5, wherein the DAC value is based on a number of bits of the SAR based ADC.

7. The method of claim 6, wherein the DAC value includes a settling time of a light-emitting diode.

8. An apparatus comprising:
a digital-to-analog converter (DAC) having a DAC value;
a logic circuit coupled to the DAC;
an analog-to-digital converter (ADC) coupled to the DAC and the logic circuit, the ADC operating in a predetermined range based on an input to the DAC; and
a comparator circuit coupled to the logic circuit, the ADC, and the DAC, wherein the logic circuit and the comparator circuit initiate a conversion at a first ADC resolution when the ADC is operating within the predetermined range and incrementally change the DAC value when the ADC is not operating within the predetermined range.

9. The apparatus of claim 8, wherein changing the DAC value changes a DAC current.

10. The apparatus of claim 8 or 9, wherein incrementally changing the DAC value comprises increasing the DAC value.

11. The apparatus of any of claims 8 to 10, wherein the input to the DAC is a photodiode current.

12. The apparatus of claim 11, wherein the ADC is a successive approximation register (SAR) based ADC.

13. The apparatus of claim 12, wherein the DAC value is based on a number of bits of the SAR based ADC.

14. The apparatus of claim 13, wherein the DAC value includes a settling time of a light-emitting diode.

15. The method of claim 7, or the apparatus of claim 14, wherein the DAC value includes a conversion time of the ADC.
